# EUROPEAN PATENT APPLICATION

(11) **EP 2 248 813 A1**
(43) Date of publication of application: **10.11.2010**
(21) Application number: 09708909.8
(22) Date of filing: 06.02.2009
(51) Int. Cl.: C07D 473/10, A61K 31/522, A61P 9/08, A61P 9/12, A61P 25/26

(54) **PRODUCTION METHOD AND PRODUCTION DEVICE FOR COMPOSITION HAVING HIGH CONTENT OF THEOBROMINE**

(30) Priority: 07.02.2008 JP 2008027944
(71) Applicant: Meiji Seika Kaisha, Ltd., Chuo-ku Tokyo 104-8002 (JP)
(72) Inventor: Satoshi HANAMURA, Saitama-ken 350-0289 (JP); Ken CHIKAUCHI, Saitama-Ken 350-0289 (JP); Shigeru KANAZAWA, Saitama-ken 350-0289 (JP); Jinichiro KOGA, Saitama-ken 350-0289 (JP); Minoru KANEGAE, Saitama-ken (JP)
(74) Representative: Grünecker, Kinkeldey, Stockmair & Schwanhäusser Anwaltssozietät
(86) International application number: PCT/JP2009/052117
(87) International publication number: WO 2009/099221

(57) **Abstract**

An object is to provide a method for producing a high theobromine-containing composition effectively in a simple way. The method for producing a theobromine-containing composition comprises: (a)performing an extraction of a theobromine-containing plant or a processed product thereof with a solvent, to obtain a crude theobromine extract; (b) applying the crude theobromine extract to a cation exchange resin subjected in advance to hydrogen ion substitution, to allow an adsorption of theobromine onto the cation exchange resin; and (c) passing a solvent containing no ionic substance through the cation exchange resin subsequently to the step (b), to obtain a theobromine eluate.

## Description

### Technical Field

The present invention relates to a high theobromine-containing composition, and a producing method thereof. More specifically, the invention relates to a method that theobromine is selectively extracted from a theobromine-containing plant or a processed product thereof to produce a high theobromine-containing composition effectively.

### Background Art

Theobromine is a natural chemical component extracted from, for example, a cacao plant. It is known that the component has various physiological effects such as a vasodilator effect, a central nervous system stimulating effect and a diuretic effect. As a typical food or drink containing theobromine, cocoa or chocolate processed from cacao, or the like is known. In recent years, research on physiological effects, such as a relaxing effect, based on such food or drink has been advancing, and in the situation a functional food the theobromine content is made high has been expected to be commercialized. Thus, if a high theobromine-containing composition can be prepared from cacao or other natural plants, the addition of the present composition to a food or drink makes it possible to give the physiological effects of theobromine thereto even if the food or drink which containing no theobromine. Japanese Patent Application National Publication No. 2004-536792 (Patent Document 1) discloses a method for obtaining a composition containing a specified amount of theobromine from cacao.

Patent Document 1: JP-A-2004-536792
Non-Patent Document 1: AOAC Official Methods of Analysis (1990) 980. 14, "Theobromine and Caffeine in Cacao Products Liquid Chromatographic Method"

### Disclosure of the Invention

However, the method disclosed in Patent Document 1 has a large number of steps; thus, it is difficult to say that the method is simple and effective to collect theobromine. Furthermore, Patent Document 1 discloses a method of separating and purifying a theobromine fraction by ion exchange chromatography. In this method, an acidic solution is used to elute out the theobromine fraction from an ion exchange chromatographer. It is however known that a theobromine fraction solution obtained by this method is decomposed in the step of powderization since the solution is acidic. Even if the acidic solution is neutralized with an alkaline solution in this method, a subsequent desalting step becomes necessary so that the number of steps increases. Furthermore, there is caused a problem that the desalting step causes a fall in the recovery of theobromine.

Accordingly, an object of the invention is to provide a method making it possible to produce a high theobromine-containing composition simply and effectively. In light of the above-mentioned situation, the inventors have made eager investigations to find out that when a theobromine-containing crude extract is applied to a cation exchange resin subj ected in advance to hydrogen ion substitution, theobromine is effectively adsorbed on the resin and unnecessary other components are eluted out. The invention is based on this finding, and has, as features thereof, subject matters described in the following:

(1) A method for producing a theobromine-containing composition, comprising the following steps:
(a) performing an extraction of a theobromine-containing plant or a processed product thereof with a solvent, to obtain a crude theobromine extract;
(b) applying the crude theobromine extract to a cation exchange resin subjected in advance to hydrogen ion substitution, to allow an adsorption of theobromine onto the cation exchange resin; and
(c) passing a solvent containing no ionic substance through the cation exchange resin subsequently to the step (b), to obtain a theobromine eluate.

(2) The producing method according to item (1), wherein, the solvent containing no ionic substance is a solution of an organic solvent in water, or deionized water in the step (c).

(3) The producing method for yielding a theobromine-containing composition according to item (1) or (2), wherein the step (c) comprises the steps: (c1) passing a first solvent therethrough; and (c2) passing a second solvent therethrough subsequently to the step (c1).

(4) The producing method according to item (3), wherein the second solvent is a 30-95% by weight aqueous ethanol solution.

(5) The producing method according to item (3), wherein the second solvent is deionized water having a temperature of 40 to 100°C.

(6) The producing method according to any one of items (3) to (5), wherein the first solvent is deionized water having a temperature of 35°C or lower.

(7) The producing method according to any one of items (1) to (6), wherein the solvent used for the extraction is a 0-80% by weight aqueous ethanol solution in the step (a).

(8) The producing method according to any one of items (1) to (7), wherein the theobromine-containing plant is a cacao.

(9) The producing method according to any one of items (1) to (8), wherein the step (b) is performed by filling the cation exchange resin into a column and passing the crude theobromine extract at a flow rate SV of 2 to 10 therethrough.

(10) The producing method according to any one of items (1) to (9), further comprises a step (d) concentrating or drying the theobromine eluate obtained in the step (c).

(11) A theobromine-containing composition yielded by the producing method according to any one of items (1) to (10).

(12) An apparatus for producing a theobromine-containing composition from a theobromine-containing plant or a processed product thereof, comprising:
an extraction section having a means for obtaining a crude theobromine extract from a theobromine-containing plant or a processed product thereof;
a purification section having a means for obtaining a theobromine eluate by applying the crude theobromine extract transferred through a means for transferring from the extraction section to a cation exchange resin subjected in advance to hydrogen ion substitution; and
a recovery section having a means for concentrating or drying the theobromine eluate transferred through the means for transferring from the purification section.

The present application claims the priority based on Japanese Patent Application No. 2008-27944 filed on February 7, 2008, the contents of which are incorporated into this specification by reference to the specification of the earlier filed application.

According to the producing method of the invention, a high theobromine-containing composition can be effectively produced in a simple way. The invention has advantages that the steps are simple, the recovery of theobromine is high and the amount of the solvent to be used is small since it is unnecessary to subject the crude extract in advance to treatment with a porous resin or the like.

In general, an ionic solution is used for the elution of a target substance from an ion exchange chromatograph. However, according to the invention, theobromine can be eluted out with a solvent containing no ionic substance; therefore, steps after the elution can be made simple so that inestimable economic advantage is produced.

Furthermore, the composition obtained by the invention has a high theobromine content; thus, the composition is easily blended with food, drink, a medical supply, or the like, as an additive. Moreover, the blend amount thereof is also easily adjusted.

### Brief Description of the Drawings

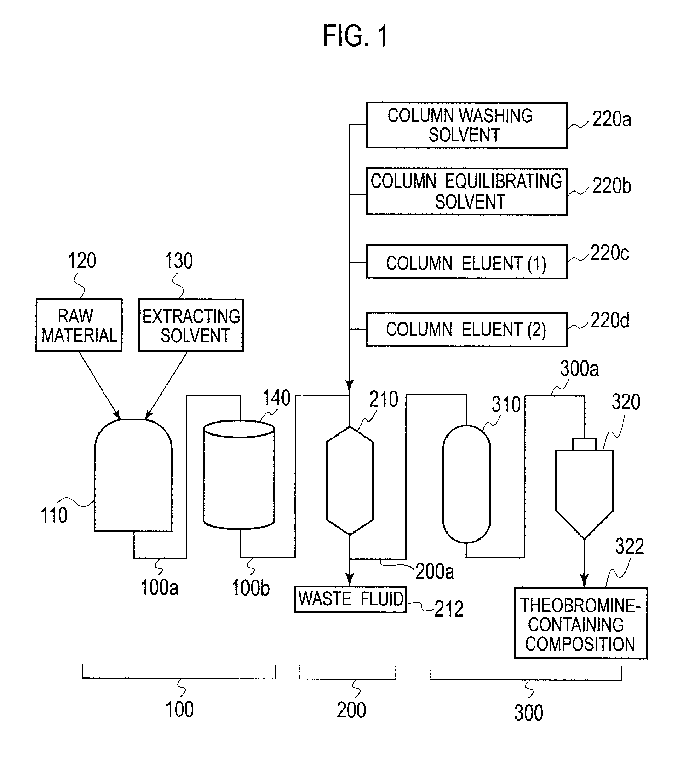

[FIG. 1] FIG. 1 is a flowchart describing an embodiment of the producing apparatus of the invention.

### Reference Numerals

- 100:: Extraction section
- 100a:: Pipe
- 110:: Extracting tank
- 120:: Raw material tank
- 130:: Solvent tank
- 140:: Centrifuge
- 200:: Purification section
- 200a:: Pipe
- 210:: Column
- 212:: Waste tank
- 220a,: 220b, 220c, and 220d: Solvent tanks
- 300:: Recovery section
- 300a:: Pipe
- 310:: Concentrator
- 320:: Spray drier
- 322:: Collecting tank

### Best Mode for Carrying Out the Invention

Hereinafter, the invention will be described in detail. A first of the invention relates to a method for producing a high theobromine-containing composition, and this producing method has a step (a) of performing an extraction of a theobromine-containing plant or a processed product thereof with a solvent, to obtain a crude theobromine extract; a step (b) of applying the crude theobromine extract to a cation exchange resin subjected in advance to hydrogen ion substitution, and thereby allowing an adsorption of theobromine onto the cation exchange resin; and a step (c) of passing a solvent containing no ionic substance through the cation exchange resin subsequently to the step (b), to obtain a theobromine eluate. The term "theobromine-containing plant or a processed product thereof" used in the present specification denotes any plant or any processed product thereof that contains, as a component thereof, theobromine, and means that no especial limitation is imposed onto one or more components coexisting with.

Examples of the raw material usable in the invention, which is not particularly limited, include cacao, Ilex paraguariensis(Yerba mate), Camellia sinensis(tea tree), kola, guarana and Coffea(coffee tree) ; and processed products thereof. Of these materials, preferred is a cacao plant or a processed product thereof, which is easily available and has relatively high theobromine content. Cacao contains not only theobromine but also polyphenols, which is useful as a physiological effect component. Thus, a case where a cacao plant or a processed product thereof is used as the raw material in the producing method of the invention is preferred also from a viewpoint that a fraction rich in cacao polyphenol can be obtained in the step of separating theobromine.

The term "high theobromine-containing composition" used in the specification means that the theobromine component content in the solid content thereof is 30% or more by weight, more preferably 40% or more by weight.

The following will describe the method for producing a theobromine-containing composition according to the invention in detail, giving, as an example, the case of using a cacao plant or a processed product thereof as a raw material. It is however needless to say that the producing method according to the invention can be carried out in the same way even when plants other than cacao, and processed products thereof are each used as a raw material. The "cacao plant or the processed product" usable in the invention may be, for example, a plant such as cacao beans, cacao shells; or a processed product of cacao beans such as cacao liquor, defatted cacao liquor, or cocoa powder. Cacao liquor is obtained by grinding cacao beans, and defatted cacao liquor is obtained by removing fat from cacao liquor. The method for removing fat from cacao liquor is not particularly limited, and may be a known method such as squeezing. Cocoa powder can be obtained by pulverizing defatted cacao liquor. Of the raw materials given as the examples, cacao liquor and cocoa powder are preferred since these are subjected, in a processing method therefor, to finely-granulating such as grinding, and pulverizing so that theobromine can be effectively extracted.

In the producing method of the invention, the step (a) is concerned with the step of obtaining a crude theobromine extract from a theobromine-containing raw material. No especial limitation is imposed to this step as far as extraction is conducted with a solvent. A well-known extracting technique may be applied thereto. The technique may be appropriately selected from well-known solvent extraction methods, for example, a method of putting the raw material into an extracting kettle, and soaking the material in a predetermined amount of an extracting solvent for a specified period to obtain an extract, and a method of sending an extracting solvent to the raw material filled into a column to obtain a predetermined amount of an extract. Components insoluble in the extracting solvent (residues) are removed by centrifugation, filtration, or some other method. The thus obtained crude theobromine extract may be a solution obtained by the extracting and not subjected to any subsequent treatment, wherein the solvent is contained in a large amount, or a solution wherein the extracting solvent is partially distilled off.

When a cacao plant or a processed product thereof is used as a raw material, a crude cacao extract is obtained from the solvent extraction in the step (a). The solvent used for the extraction may be water, an organic solvent such as ethanol, methanol, acetonitrile, and an aqueous solution thereof. Although the extracting solvent is not particularly limited, it is preferred to use water, ethanol, and an aqueous solution thereof since the solvents are widely usable in the production of food. These solvents are preferred also since the solvents are harmless to human. As the solvents, ethanol may be used alone; more preferably, water, or a mixture of water and ethanol, that is, an aqueous ethanol solution is used. The ethanol content in the aqueous ethanol solution is preferably from 0 to 95% by weight, more preferably from 30 to 95% by weight, even more preferably from 40 to 80% by weight. The solvent used in the step (a) is most preferably a 50% by weight aqueous ethanol solution.

The temperature at the time of the extraction ranges theoretically from 0 to 100°C, preferably from 50 to 90°C in the case of using, for example, water as the extracting solvent. In the case of using an aqueous ethanol solution as the extracting solvent, the temperature ranges theoretically from 0 to 80°C, preferably from 40 to 70°C. It is advisable to decide the extracting period appropriately under the consideration of the used raw material, and other extracting parameters. The extracting period, which is not particularly limited, is usually from about 10 to 60 minutes. The crude cacao extract obtained as described above contains cacao polyphenol, amino acids, saccharides, colorants, fats, and other unnecessary components in a large amount together with theobromine.

The step (b) in the producing method of the invention is a step of applying the crude theobromine extract obtained from the precedent step (a) to a cation exchange resin subjected in advance to hydrogen ion substitution, to allow an adsorption of theobromine onto the cation exchange resin. The term "cation exchange resin" used in the specification means a strongly acidic or weakly acidic cation exchange resin, which is well known and is not particularly limited. The "cation exchange resin in advance subjected to hydrogen ion substitution" denotes that the cations in the resin, which are to be reactive groups, are in advance subjected to substitution with hydrogen ions.

In the invention, specific usable examples of the cation exchange resin include DIAION (registered trade name) series SK1B and SK110, manufactured by Mitsubishi Chemical Corp., AMBERLITEs (registered trade name) IR-120B, IR-200CT and IRC50, manufactured by Rohm and Haas Co., and DOWEX (registered trade name) 50W-X8 manufactured by the Dow Chemical Co. The substitution of the cation exchange resin with hydrogen ions can be conducted, for example, by applying an acid having an appropriate concentration to the resin. In the substitution treatment, for example, 1 N hydrochloric acid may be used. In the invention, it appears that theobromine is more easily adsorbed onto the cation exchange resin by subjecting the cation exchange resin in advance to hydrogen ion substitution. This matter has been investigated in Examples, which will be described later. The cation exchange resin used in the invention is not particularly limited, however, when any resin is used, it is essential to subject the resin in advance to hydrogen ion substitution.

In the step (b), for the contact of the cation exchange resin with the crude theobromine extract, more specifically the crude cacao extract, any method may be used as far as the method is capable of an adsorption of theobromine in the crude cacao extract effectively onto the cation exchange resin. The step (b) in the invention may be carried out, for example, by filling a cation exchange resin subjected in advance to substitution with hydrogen ions into a column, and next passing the crude cacao extract through the cation exchange resin in the column. In another method, the step (b) can be carried out by putting a cation exchange resin subjected in advance to hydrogen ion substitution directly into the crude cacao extract and then stirring the mixture. When such a method is used, it is necessary to allow an adsorption of theobromine onto the cation exchange resin and subsequently recovery the cation exchange resin by filtration or some other separating method. In other words, the latter method is a batch treatment, and thus the column-used method is more preferred from the viewpoint of work efficiency.

Conditions for the applying the crude cacao extract to cation exchange resin may be appropriately selected in accordance with the concentration in the crude cacao extract, and others. For example, when a column is used to conduct the treatment continuously, it is preferred to pass, through the column into which the cation exchange resin (the resin substituted in advance with hydrogen ions) is filled, the crude cacao extract having a volume 1-20 times the amount of the filled resin at a flow rate SV of about 1 to 10, preferably about 2 to 10. When the column-used purification is conducted, it becomes substantially necessary to use a solvent for equilibrating the cation exchange resin. In the invention, as the equilibrating solvent, deionized water or an aqueous ethanol solution having an ethanol content of 0 to 50% by weight, more preferably 0 to 10% by weight, is used. After the cation exchange resin is equilibrated, the crude cacao extract is passed through the resin, and subsequently a solvent containing no ionic substance is passed therethrough, thereby making it possible to obtain an eluate having higher theobromine content. Substantially, by passing a solvent containing no ionic substance through the resin as described below, dissociation and elution of theobromine from the cation exchange resin are attained.

The step (c) in the producing method of the invention relates to the step of passing a solvent containing no ionic substance through the cation exchange resin subsequently to the step (b), and thereby a theobromine eluate is provided. In other words, in the producing method of the invention, the step (c) is substantially a separating and purifying step for obtaining a fraction containing theobromine at a high concentration. In general, for the elution of a target substance from an ion exchange chromatograph which uses a cation exchange resin or the like is used as a filler, an ionic solution is used which contains a substance capable of attaining ion exchange for the ion exchange resin, examples of the solution including respective ionic solutions of hydrochloric acid, sodium hydroxide, calcium chloride, and sodium chloride. However, in the producing method according to the invention, the elution of theobromine from the cation exchange resin is carried out by passing a solvent containing no ionic substance through the resin. By such a combination of the use of a cation exchange resin subjected in advance to substitution with hydrogen ions with a solvent containing no ionic substance, theobromine can be obtained at a high recovery from a raw material in the invention, so that a high theobromine-containing composition can be simply and effectively supplied.

It is preferred to pass the solvent containing no ionic substance through the resin at a flow rate SV of about 1 to 10, preferably about 2 to 10. By passing the solvent therethrough at such a flow rate, it becomes easy to obtain a composition (in a solution state) the theobromine content in solid content is high. About the flow rate, for example, an "SV of 1" means that a liquid is caused to flow in a volume equivalent to (as much as) the resin volume per hour. The term "solvent containing no ionic substance" used in the specification means a solvent which does not contain any substance that is adsorbed onto or desorbed from a cation exchange resin. Examples of the solvent include deionized water and an aqueous solution of an organic solvent.

When deionized water is used for the elution of theobromine, the temperature of deionized water ranges from 40 to 100°C, preferably from 40 to 90°C, more preferably from 60 to 80°C. When deionized water 40°C or lower in temperature is used, it tends to become difficult to elute out theobromine adsorbed on the resin. In the meantime, the aqueous solution of organic solvent is as exemplified above about the step (a). The most preferred solvent is an aqueous ethanol solution. The ethanol content in the aqueous ethanol solution is preferably from 0 to 95% by weight, more preferably from 30 to 95% by weight, even more preferably from 40 to 80% by weight. The aqueous solution of organic solvent used in the step (c) is most preferably a 50% by weight aqueous ethanol solution.

In order to separate theobromine and any other component contained in the crude theobromine extract from each other, it is preferred to select two solvent species appropriately and use the species in the step (c). For example, a crude cacao extract contains, besides theobromine, cacao polyphenol, amino acids, saccharides, colorants, fats, and other components in a large amount. Theobromine is more easily adsorbed on the cation exchange resin than the other components, such as cacao polyphenol; thus, when the crude cacao extract is passed through the cation exchange resin, the other components, such as the polyphenol, are earlier eluted out. This matter makes it possible to elute out the other components, such as cacao polyphenol, selectively while a theobromine fraction is adsorbed on the resin, and further elute out theobromine more selectively by removing the other component fraction and then eluting out the theobromine fraction.

Accordingly, in an embodiment of the producing method according to the invention, the step (c) preferably has a step (c1) of passing a first solvent through the resin, and a step (c2) of passing a second solvent therethrough subsequently to the step (c1). The second solvent is substantially a solvent for eluting out theobromine. As exemplified above, the second solvent may be, for example, deionizied water having a temperature of 40 to 100°C, or an aqueous solution of an organic solvent, such as an aqueous ethanol solution. In the meantime, the first solvent may be any solvent that causes theobromine to be adsorbed on the cation exchange resin but makes it possible to elute out the other components. From the viewpoint of the adsorptivity of theobromine onto the resin, the first solvent may be deionized water having a temperature lower than 40°C, or an aqueous solution of organic solvent which is lower in concentration than the organic solvent used as the extracting solvent. More specifically, the aqueous solution of organic solvent may be an aqueous ethanol solution having an ethanol concentration of 30% or less by weight, more preferably 10% or less by weight. Considering costs, the solution is most preferably deionized water preferably having a temperature of 35°C or lower, more preferably having 25°C or lower.

In an example of the embodiment, the first solvent, which is not particularly limited, is preferably deionized water having a temperature of 35°C or lower. When deionized water having a temperature higher than 35°C is used, theobromine tends to be easily eluted out together with the other components, such as cacao polyphenol. In the meantime, it is preferred that the second solvent is deionized water having a temperature higher than 35°C and is more preferably deionized water having a temperature of from 40°C or higher to lower than 100°C, or an aqueous solution of organic solvent. Details of the deionized water or the aqueous solution of organic solvent usable as the second solvent is as described above.

By selecting the solvents to be passed through the resin appropriately as described above, applying the crude cacao extract to the cation exchange resin and then using the individual solvents in turn, the unnecessary components, such as cacao polyphenol, can be washed away by use of the deionized water 35°C or lower in temperature (the first solvent) in the state that theobromine is adsorbed onto the resin. Subsequently, by eluting out theobromine by use of the higher-temperature deionized water or the aqueous ethanol solution (the second solvent), an eluate rich in theobromine can be obtained. The theobromine content in the solid content obtained by removing the solvents and other fluid contents from this eluate is remarkably higher the theobromine content in the raw material. Therefore, according to the producing method, a composition having a higher theobromine content can be supplied by using, as a raw material, a theobromine-containing plant or a processed product thereof.

As the need arises, the high theobromine-containing composition that is in a solution state may be concentrated or dried. It is therefore preferred that the high theobromine-containing composition producing method of the invention has, besides the steps (a) to (c), the step (d) of concentrating or drying the theobromine eluate obtained through the step (c). By setting up this step, a solid-form high theobromine-containing composition can easily be obtained. The method for the concentration or the drying is not particularly limited, and a known method may be used. An example of the method for the concentration is concentration under reduced pressure or heating concentration. An example of the method for the drying is spray drying or freeze-drying.

A second of the invention relates to a high theobromine-containing composition obtained by the producing method according to the first of the invention. The composition obtained by the invention may be used for various purposes or articles for which theobromine is effective since the composition has a high theobromine content. It is known that theobromine has, for example, various pharmaceutical effects; thus, the composition according to the invention can easily be incorporated into a medical supply, food, drink, and or the like. More specifically, when the high theobromine-containing composition according to the invention is blended with an excipient, a stabilizer, a flavoring agent, and others that may each be of various types and may be used in ordinary drugs, a medical supply can be provided, examples of the supply comprising a tablet, a capsule agent, a granule, and a powder that each have a pharmaceutical effect. When the composition according to the invention is incorporated into a food or drink, such as cocoa, coffee, chocolate, biscuit, snack, candy, tablet sweets, gum, gummy candy, jelly, sweetened and jellied bean paste(youkan), ice cream, sherbet, beverage, dairy products, bread, sausage and ham, pharmaceutical effects can be given for them. When the use of the composition in food, drink, or a medical supply is considered, the composition according to the invention is preferably a composition the theobromine content in solid content is 30% or more by weight, more preferably 40% by weight. The producing method of the invention makes it possible to supply such a desired composition, wherein the theobromine content is high.

A third of the invention relates to an apparatus for producing a theobromine-containing composition from a theobromine-containing plant or a processed product thereof. The producing apparatus of the invention has an extraction section having a means for obtaining a crude theobromine extract from a theobromine-containing plant or a processed product thereof; a purification section having a means for obtaining a theobromine eluate by applying the crude theobromine extract transferred through a means for transferring from the extraction section to a cation exchange resin subjected in advance to hydrogen ion substitution; and a recovery section having a means for concentrating or drying the theobromine eluate transferred through a means for transferring from the purification section.

The extraction section is equipped with at least a container that can hold the above-mentioned plant or the processed product, which may be referred to as the raw material hereinafter, and a solvent used in an extraction therefrom. The extraction is conducted by causing the raw material and the solvent to contact each other in the container, and the insoluble components (residues) are removed by a removing means, and thereby a crude theobromine extract is obtained. The container preferably has at least one opening to pour the raw material and the extracting solvent, and an opening to discharge the crude theobromine extract. The container may be, for example, a column, an extracting kettle, or a tank.

In order to make the efficiency of the extraction high, a stirring means such as a propeller and a screw may be arranged in the container. A means for pulverizing the raw material into fine pieces, such as a rotating blade, may also be arranged in the container. The insoluble component removing means may be, for example, a centrifuge, and a filtrating apparatus set up outside the container. By adding a concentrating means such as an evaporator and a reduced-pressure concentrator to the extraction section as the need arises, the solvent in the crude extract can be distilled off before the extract is transferred to the purification sections, so that the liquid amount can be decreased.

The crude theobromine extract obtained in the extraction section is transferred to a purification section through a means for transferring. The means for transferring may be, for example, a liquid-sending pump and a pipe. As the need arises, a control valve may be set to the pipe. By the liquid-sending pump and the control valve, the flow quantity and the flow rate of the crude theobromine extract can be adjusted when the extract is transferred to the purification section.

The purification section has at least one column holding a cation exchange resin subjected in advance to hydrogen ion substitution, and a first solvent tank holding a first solvent containing no ionic substance. By passing the crude theobromine extract transferred from the extraction section through the resin held in the column, and subsequently further passing the first solvent, which contains no ionic substance, supplied from the first solvent tank, an eluate containing unnecessary components other than theobromine is obtained. When the crude theobromine extract is passed through the resin, theobromine is easily adsorbed on the resin so that the unnecessary components other than theobromine are earlier eluted out with ease. It is therefore preferred that the purification section has a second solvent tank holding a second solvent which contains no ionic substance and is different from the first solvent in order to conduct the separation and purification of theobromine effectively. When the first and second solvents are appropriately selected, the unnecessary components can be eluted out effectively by the passing of the first solvent through the resin, and removed.

As described above, a recovery section may be separately set up to transfer the eluate containing unnecessary components other than theobromine. This recovery section is equipped with a container for collecting the eluate containing unnecessary components, and a concentrating means for concentrating the eluate, such as a pressure-reduced concentrator. In order that after the formation of a concentrate of the eluate in this recovery section, the concentrate can be again used in the purification section, the apparatus may have a means for transferring, such as a liquid-sending pump and a pipe. After the unnecessary components are removed in this way, the second solvent is passed through the resin, and thereby an eluate rich in theobromine is provided.

The purification section may have third and fourth solvent tanks holding hydrochloric acid for subjecting a cation exchange resin to hydrogen ion substitution, a solution of sodium hydroxide that is used to wash the resin, and some other treating solution. Furthermore, a solvent tank holding a solvent to be supplied to the column may be added to as the need arises. After the theobromine eluate is obtained, by passing the treating solutions from the third and fourth solvent tanks into the column, the resin in the column is washed and regenerated, so that the purification can be continuously conducted. The supply of the solvent from each of the first to fourth solvent tanks to the column is attained by a means for transferring, such as a liquid-sending pump and a pipe. The means for transferring may contain a control valve as the need arises. By the liquid-sending pump and the control valve, the flow quantity and the flow rate of the solvent supplied to the column may be adjusted.

The theobromine eluate obtained in the purification section is transferred through a means for transferring to a recovery section. The means for transferring may be, for example, a liquid-sending pump and a pipe. As the need arises, a control valve may be set to the pipe. By the liquid-sending pump and the control valve, the flow quantity and the flow rate of the theobromine eluate can be adjusted when the eluate is transferred to the recovery section.

The recovery section has a container holding the transferred the theobromine eluate, and a means for removing the solvent in the eluate from the container. The solvent removing means may be, for example, an apparatus used for ordinary concentration, for example, a reduced-pressure concentrator, or an apparatus used for ordinary drier, for example, a freeze drier, or spray drier.

An embodiment of the producing apparatus of the invention is illustrated in FIG. 1. As illustrated in FIG. 1, the producing apparatus of the invention has an extraction section 100, a purification section 200, and a recovery section 300. The extraction section 100 has an extracting tank 100 for conducting an extraction from the raw material, a raw material tank 120 for holding the raw material, a solvent tank 130 holding an extracting solvent, and a centrifuge 140 for removing insoluble components after the extraction. The extracting tank 110 and the centrifuge 140 are connected to each other through a pipe 100a.

The purification section 200 has a column 210 for subjecting an extract transferred through a pipe 100b from the extraction section 100 to purification, a solvent tank 220a for holding a solvent for washing of the column, a solvent tank 220b for holding a solvent for equilibrating of the column, and solvent tanks 220c and 220d each for holding a solvent used for elution. Reference number 212 represents a waste tank for holding a waste.

The recovery section 300 has a concentrator 310 for concentrating an eluate transferred through a pipe 200a from the purification section 200, and a spray drier 320 for drying the concentrated liquid. The concentrator 310 and the spray drier 320 are connected to each other through a pipe 300a. Reference number 322 represents a collecting tank holding a composition obtained by the drying.

According to the producing apparatus of the invention having such a structure, a theobromine-containing plant or a processed product thereof is used as a raw material to make it possible to obtain a high theobromine-containing composition effectively. The producing apparatus of the invention is intended to be used to carry out the producing method of the invention described above. Accordingly, the solvent used in each of the sections, the resin held in the column and the other various conditions are equivalent to the various conditions in the producing method.

### Examples

Hereinafter, the invention will be specifically described by way of working examples; however, the invention is not limited to these working examples.

The theobromine content described in the individual working examples and individual comparative examples are values obtained by measurements according to the following method:
(Theobromine content)
The theobromine content were each a value obtained by using commercially available theobromine as a standard product, and quantitating the solid content of the composition obtained in any one of the individual working examples and the individual comparative examples with reference to a method described in AOAC Official Methods of Analysis (1990) 980. 14, "Theobromine and Caffeine in Cacao Products Liquid Chromatographic Method".

The method for the analysis is specifically as follows:
First, each of the samples was precisely weighed into a centrifugal tube, and 30 mL of petroleum ether was added. The mixture was sufficiently stirred, and then centrifuged. The supernatant was thrown away. The defatted sample was transferred into a conical flask, and water was added to fill up to about 100 mL. This solution was heated in 100°C boiled water for 25 minutes. After the heating, the solution was immediately cooled, and 10 mL of a 2% by weight aqueous zinc sulfate solution and 10 mL of a 1.8% by weight barium hydroxide were added. The components were mixed with each other and then the mixture was allowed to stand still. Water was added to this solution to make the solution into a fixed volume of 200 mL, and the solution was again heated in 100°C boiled water for 10 minutes. After the heating, the solution was filtrated to prepare a sample solution. The thus obtained sample solution was used to make a measurement by high-performance liquid chromatography. Conditions for the high-performance liquid chromatographic measurement were as follows:

Column for the analysis: Waters µ-Bondapk C18 10 µm 4 mmID × 300 mm (or an equivalent thereto)
Mobile phase: water and acetonitrile (85:15)
Mobile phase flow rate: 1.0 mL/minute, and Detection: UV 273 nm

### (Example 1)

Cacao beans (dried in the production area thereof; the theobromine content: 1.3% by weight) were used to carry out the following steps (a) to (d), and thereby prepare a theobromine-containing composition:
(a) One hundred grams of the cacao beans were ground. To the ground cacao beans was added a 50% by weight aqueous ethanol solution. The mixture was stirred at 50°C for 30 minutes, and then the insoluble components were removed by centrifugation to obtain a crude cacao extract.
(b) Next, the crude cacao extract was passed at a flow rate SV of 5 through a column into which 1000 mL of a cation exchange resin (AMBERLITE IR-120B) subjected in advance to hydrogen ion substitution was filled.
(c1) Subsequently, 20°C deionized water was passed through the column to wash away components not adsorbed onto the cation exchange resin.
(c2) A 50% by weight aqueous ethanol solution was passed through this column to obtain an eluate.
(d) This eluate was freeze-dried to obtain a theobromine-containing composition.
The theobromine content in the solid content of the composition was analyzed. As a result, the content was 73.9% by weight. Separately, a crude cacao extract obtained by carrying out the step (a) was freeze-dried and the theobromine content in the solid content thereof was analyzed. The content was 8.5% by weight.

### (Example 2)

Cacao beans (dried in the production area thereof; the theobromine content: 1.3% by weight) were used to carry out the following steps (a) to (d), and thereby prepare a theobromine-containing composition:
(a) One hundred grams of the cacao beans were ground. To the ground cacao beans was added a 50% by weight aqueous ethanol solution. The mixture was stirred at 50°C for 30 minutes, and then the insoluble components were removed by centrifugation to obtain a crude cacao extract.
(b) Next, the crude cacao extract was passed at a flow rate SV of 5 through a column into which 1000 mL of a cation exchange resin (AMBERLITE IR-120B) subjected in advance to hydrogen ion substitution was filled.
(c1) Subsequently, 35°C deionized water was passed through the column to wash away components not adsorbed onto the cation exchange resin.
(c2) A 95% by weight aqueous ethanol solution was passed through this column to obtain an eluate.
(d) This eluate was freeze-dried to obtain a theobromine-containing composition.
The theobromine content in the solid content of the composition was analyzed. As a result, the content was 41.5% by weight.

### (Example 3)

Cocoa powder (the fat content: 12% by weight, and the theobromine content: 2.0% by weight) was used to carry out the following steps (a) to (d), and thereby prepare a theobromine-containing composition:
(a) One hundred grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 100 mL of a cation exchange resin (DOWEX 50W-X8) subjected in advance to hydrogen ion substitution was filled.
(c1) Subsequently, 25°C deionized water was passed through the column to wash away components not adsorbed onto the cation exchange resin.
(c2) A 30% by weight aqueous ethanol solution was passed through this column to obtain an eluate.
(d) This eluate was freeze-dried to obtain a theobromine-containing composition.
The theobromine content in the solid content of the composition was analyzed. As a result, the content was 45.7% by weight. Separately, a crude cacao extract obtained by carrying out the step (a) was freeze-dried and the theobromine content in the solid content thereof was analyzed. The content was 3.3% by weight.

### (Example 4)

Cocoa powder (the fat content: 12% by weight, and the theobromine content: 2.0% by weight) was used to carry out the following steps (a) to (d), and thereby prepare a theobromine-containing composition:
(a) One hundred grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 500 mL of a cation exchange resin (AMBERLITE IR-120B) subjected in advance to hydrogen ion substitution was filled.
(c1) Subsequently, 25°C deionized water was passed through the column to wash away components not adsorbed onto the cation exchange resin.
(c2) 80°C deionized water was passed through this column to obtain an eluate.
(d) This eluate was freeze-dried to obtain a theobromine-containing composition.
The theobromine content in the solid content of the composition was analyzed. As a result, the content was 48.2% by weight.

### (Example 5)

Cocoa powder (the fat content: 12% by weight, and the theobromine content: 2.0% by weight) was used to carry out the following steps (a) to (d), and thereby prepare a theobromine-containing composition:
(a) One hundred grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 500 mL of a cation exchange resin (AMBERLITE IR-120B) subjected in advance to hydrogen ion substitution was filled.
(c1) Subsequently, 30°C deionized water was passed through the column to wash away components not adsorbed onto the cation exchange resin.
(c2) 40°C deionized water was passed through this column to obtain an eluate.
(d) This eluate was freeze-dried to obtain a theobromine-containing composition.
The theobromine content in the solid content of the composition was analyzed. As a result, the content was 40.5% by weight.

### (Comparative Example 1)

A theobromine-containing composition was prepared in the same way as in Example 1 except that the step (c1) in Example 1 was not conducted. The method is specifically as follows:
(a) One hundred grams of the same cacao beans as in Example 1 were ground. To the ground cacao beans was added a 50% by weight aqueous ethanol solution. The mixture was stirred at 50°C for 30 minutes, and then the insoluble components were removed by centrifugation to obtain a crude cacao extract.
(b) Next, the crude cacao extract was passed at a flow rate SV of 5 through a column into which 1000 mL of a cation exchange resin (AMBERLITE IR-120B) subjected in advance to hydrogen ion substitution was filled.
(c2) Subsequently, a 50% by weight aqueous ethanol solution was passed through this column to obtain an eluate.
(d) This eluate was freeze-dried to obtain a theobromine-containing composition.
The theobromine content in the solid content of the composition was analyzed. As a result, the content was 3.5% by weight. From the results, it is understood that by passing deionized water having a low temperature (35°C or lower) through the resin before the elution of theobromine, theobromine is more easily extracted.

### (Comparative Example 2)

Cocoa powder (the fat content: 12% by weight, and the theobromine content: 2.0% by weight) was used to carry out the following steps (a) to (d), and thereby prepare a theobromine-containing composition:
(a) Ten grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 20 mL of a cation exchange resin (AMBERLITE IR-120B) subjected in advance to hydrogen ion substitution was filled.
(c1) Subsequently, 40°C deionized water was passed through the column to wash away components not adsorbed onto the cation exchange resin.
(c2) A 50% by weight aqueous ethanol solution was passed through this column to obtain an eluate.
(d) This eluate was freeze-dried to obtain a theobromine-containing composition.
The theobromine content in the solid content of the composition was analyzed. As a result, the content was 4.5% by weight. Separately, a crude cacao extract obtained by carrying out the step (a) was freeze-dried and the theobromine content in the solid content thereof was analyzed. The content was 3.3% by weight. From the results, it is thinkable that when 35°C or higher deionized water is passed through the cation exchange resin in the step (c1), theobromine is not easily adsorbed onto the resin. In other words, it has been found out that theobromine is eluted out together with other components and theobromine tends not to be easily separated or eluted out.

### (Comparative Example 3)

Cocoa powder (the fat content: 12% by weight, and the theobromine content: 2.0% by weight) was used to carry out the following steps (a) to (d), and thereby prepare a theobromine-containing composition:
(a) One hundred grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 1000 mL of a cation exchange resin (AMBERLITE IRA-96SB) subjected in advance to hydroxide ion substitution was filled.
(c1) Subsequently, 25°C deionized water was passed through the column to wash away components not adsorbed onto the cation exchange resin.
(c2) A 50% by weight aqueous ethanol solution was passed through this column to obtain an eluate.
(d) This eluate was freeze-dried to obtain a theobromine-containing composition.
The theobromine content in the solid content of the composition was analyzed. As a result, the content was 0.4% by weight. From the results, it has been found out that when an anion exchange resin is used instead of a cation exchange resin, a selective elution of theobromine is difficult.

### (Comparative Example 4)

Cocoa powder (the fat content: 12% by weight, and the theobromine content: 2.0% by weight) was used to carry out the following steps (a) to (d), and thereby prepare a theobromine-containing composition:
(a) Ten grams of the cocoa powder was dispersed into water. The mixture was stirred at 90°C for 30 minutes, and then the insoluble components were removed by centrifugation to obtain a crude cacao extract.
(b) Next, the crude cacao extract was passed at a flow rate SV of 5 through a column into which 20 mL of a cation exchange resin (AMBERLITE IR-120B) subjected in advance to sodium ion substitution was filled.
(c1) Subsequently, 25°C deionized water was passed through the column to wash away components not adsorbed onto the cation exchange resin.
(c2) A 50% by weight aqueous ethanol solution was passed through this column to obtain an eluate.
(d) This eluate was freeze-dried to obtain a theobromine-containing composition.
The theobromine content in the solid content of the composition was analyzed. As a result, the content was 0.3% by weight. From the results, it has been found out that when a cation exchange resin not subjected to hydrogen ion substitution is used, it is difficult to separate and elute out theobromine selectively. In other words, cation exchange resin not subjected to hydrogen ion substitution adsorbs theobromine hardly.

From the above description, it is evident that the invention can be made into various and different embodiments without disobeying neither the scope nor the sprit of the invention. The invention is not limited by any specified one of the embodiments except that the invention is limited by the claims.

## Claims

1. A method for producing a theobromine-containing composition, comprising the following steps:
(a) performing an extraction of a theobromine-containing plant or a processed product thereof with a solvent, to obtain a crude theobromine extract;
(b) applying the crude theobromine extract to a cation exchange resin subj ected in advance to hydrogen ion substitution , to allow an adsorption of theobromine onto the cation exchange resin; and
(c) passing a solvent containing no ionic substance through the cation exchange resin subsequently to the step (b), to obtain a theobromine eluate.

2. The producing method according to claim 1, wherein the solvent containing no ionic substance is a solution of an organic solvent in water, or deionized water in the step (c).

3. The producing method for yielding a theobromine-containing composition according to claim 1 or 2, wherein the step (c) comprises the steps: (c1) passing a first solvent therethrough; and (c2) passing a second solvent therethrough subsequently to the step (c1).

4. The producing method according to claim 3, wherein the second solvent is a 30-95% by weight aqueous ethanol solution.

5. The producing method according to claim 3, wherein the second solvent is deionized water having a temperature of 40 to 100°C.

6. The producing method according to any one of claims 3 to 5, wherein the first solvent is deionized water having a temperature of 35°C or lower.

7. The producing method according to any one of claims 1 to 6, wherein the solvent used for the extraction is a 0-80% by weight aqueous ethanol solution in the step (a).

8. The producing method according to any one of claims 1 to 7, wherein the theobromine-containing plant is a cacao.

9. The producing method according to any one of claims 1 to 8, wherein the step (b) is performed by filling the cation exchange resin into a column and passing the crude theobromine extract at a flow rate SV of 2 to 10 therethrough.

10. The producing method according to any one of claims 1 to 9, further comprises a step (d) concentrating or drying the theobromine eluate obtained in the step (c).

11. A theobromine-containing composition yielded by the producing method according to any one of claims 1 to 10.

12. An apparatus for producing a theobromine-containing composition from a theobromine-containing plant or a processed product thereof, comprising:
an extraction section having a means for obtaining a crude theobromine extract from a theobromine-containing plant or a processed product thereof;
a purification section having a means for obtaining a theobromine eluate by applying the crude theobromine extract transferred through a means for transferring from the extraction section to a cation exchange resin subjected in advance to hydrogen ion substitution; and
a recovery section having a means for concentrating or drying the theobromine eluate transferred through the means for transferring from the purification section.
